# EUROPEAN PATENT SPECIFICATION

(11) **EP 3 636 747 B1**
(45) Date of publication and mention of the grant of the patent: **29.11.2023**
(21) Application number: 18199955.8
(22) Date of filing: 11.10.2018
(51) Int. Cl.: C12N 5/0786, A61K 35/15, G01N 33/50, A61K 35/00

(54) **CD11B+ PIEZO-1+ MACROPHAGES AND USES THEREOF**
CD11B+-PIEZO-1+-MAKROPHAGEN UND VERWENDUNGEN DAVON
MACROPHAGES CD11B + PIÉZO-1 + ET LEURS UTILISATIONS

(43) Date of publication of application: 15.04.2020
(73) Proprietor: Albert-Ludwigs-Universität Freiburg, 79085 Freiburg (DE)
(72) Inventor: Shastri, Venkatram Prasad, 79206 Breisach (DE); Sarem, Melika, 79106 Freiburg (DE)
(74) Representative: Krauss, Jan

(56) References cited:
- MA SHANG ET AL: "CommonPIEZO1Allele in African Populations Causes RBC Dehydration and AttenuatesPlasmodiumInfection", CELL, CELL PRESS, AMSTERDAM, NL, vol. 173, no. 2, 22 March 2018 (2018-03-22), page 443, XP085371348, ISSN: 0092-8674, DOI: 10.1016/J.CELL.2018.02.047
- RAJ SEWDUTH ET AL: ""Decoding" Angiogenesis: New Facets Controlling Endothelial Cell Behavior", FRONTIERS IN PHYSIOLOGY, vol. 7, 21 July 2016 (2016-07-21), XP055543945, DOI: 10.3389/fphys.2016.00306
- JING LI ET AL: "Piezo1 integration of vascular architecture with physiological force", NATURE, vol. 515, no. 7526, 10 August 2014 (2014-08-10), pages 279-282, XP055544773, London ISSN: 0028-0836, DOI: 10.1038/nature13701
- HEFIN I. RHYS ET AL: "Neutrophil Microvesicles from Healthy Control and Rheumatoid Arthritis Patients Prevent the Inflammatory Activation of Macrophages", EBIOMEDICINE, vol. 29, 1 March 2018 (2018-03-01), pages 60-69, XP055655286, NL ISSN: 2352-3964, DOI: 10.1016/j.ebiom.2018.02.003

## Description

The present invention relates to isolated mammalian CD11b+/PIEZO-1+ macrophage macrophage cells or neutrophil cells, pharmaceutical compositions comprising said cells, as well as uses thereof.

### Background of the Invention

Mechanical aspects of the cellular environment can influence cell function, and in this context hydrogels can serve as an instructive matrix.

Living cells of higher organisms reside in an environment that is mechanically and biologically well-defined by an extracellular matrix (ECM). Structural and mechanical aspects of the ECM, such as stiffness and topography, can have a substantial influence on different cell functions like cell growth or differentiation of the cells. In this context hydrogels can serve as such an "instructive" matrix.

Ma S, et al. (in: Common PIEZO1 Allele in African Populations Causes RBC Dehydration and Attenuates Plasmodium Infection. Cell. 2018 Apr 5;173(2):443-455.e12. doi: 10.1016/j.cell.2018.02.047) disclose a human gain-of-function PIEZO1 allele, E756del, present in a third of the African population. They tested whether gain-of-function *Piezol* expression in macrophages can affect *Plasmodium* infection, since macrophages have been shown to be important for both protection and pathology in malaria.

The organization of cells into tissue-like structures involves a complex interplay between soluble signals and those originating from the ECM.

Vascularization is critical for the survival of cells and necessary for the transport of signaling molecules to aid in regeneration. Vasculogenesis, as it pertains to in vitro studies, is the formation of lumens from dispersed endothelial cells (ECs), and it differs from angiogenesis where ECs sprout from an existing blood vessel or an EC monolayer.

To identify the factors that influence EC organization, several in vitro models have been established, including collagen gel, fibrin gel, and matrigel. These studies have revealed that arginine-glycine-aspartic acid (RGD) integrin binding sequence and soluble signals such as vascular endothelial growth factor (VEGF) and fibroblast growth factor-2 (FGF-2) are essential and that the mechanical aspects of the gel impact the formation of EC networks. Nevertheless, the factors that impact the organization of multiple ECs in freestanding tubular structures are not fully understood.

Jaipersad AS et al. (in: Jaipersad AS et al. The role of monocytes in angiogenesis and atherosclerosis. J Am Coll Cardiol. 2014 Jan 7-14;63(1):1-11) disclose that the two known mechanisms resulting in the formation of new vessels are local ischemia and inflammation. Blood monocytes play an important role in both processes. Monocytes are comprised of distinct subsets with different cell surface markers and functional characteristics and this heterogeneity may be relevant to angiogenic processes in atherosclerosis. The aim of the review article is to present an overview of the available evidence supporting a role for monocytes in angiogenesis and atherosclerosis.

Circulating myeloid cells can be recruited to sites of active angiogenesis and play a role in both maturation and stabilization of new vessels (Murdoch C, Muthana M, Coffelt SB, Lewis CE. The role of myeloid cells in the promotion of tumour angiogenesis. Nat Rev Cancer 2008, 8(8): 618-631).

CD11b is a marker of macrophages, neutrophils, mast cells, and microglia. The CD11 protein is actually a heterodimer complex that consists of CD11b and CD18. CD11 is involved in numerous adhesion-related associations between cells such as monocytes, macrophages, natural killer (NK) cells, and granulocytes. CD11 also regulates the uptake of complement-coated particles within cells. It is also gained usage as a microglial marker for tissues derived from the nervous system.

In particular, a specific population of CD11b+ monocytes called Neuropilin-Expressing Monocytes (NEM), as they express Neuropilin-1 (Nrp1), a co-receptor for VEGF and Semaphorin 3A co-receptor, has been recently found to accelerate new vessel stabilization, both directly by activating TGF-b1 signaling (Groppa E, Brkic S, Bovo E, Reginato S, Sacchi V, Di Maggio N, et al. VEGF dose regulates vascular stabilization through Semaphorin3A and the Neuropilin-1+ monocyte/TGF-beta1 paracrine axis. EMBO Mol Med 2015, 7(10): 1366-1384) and indirectly by promoting pericyte recruitment through PDGF-BB secretion (Zacchigna S, Pattarini L, Zentilin L, Moimas S, Carrer A, Sinigaglia M, et al. Bone marrow cells recruited through the neuropilin-1 receptor promote arterial formation at the sites of adult neoangiogenesis in mice. J Clin Invest 2008, 118(6): 2062-2075).

In order to be therapeutically useful, newly induced vascular structures must stabilize, i.e. persist long-term without regression. Newly induced vessels require about 4 weeks to become independent of further angiogenic stimuli and persist indefinitely (Ozawa CR, Banfi A, Glazer NL, Thurston G, Springer ML, Kraft PE, et al. Microenvironmental VEGF concentration, not total dose, determines a threshold between normal and aberrant angiogenesis. J Clin Invest 2004, 113(4): 516-527).

Liu et al. (in: Liu et al. Cutting Edge: Piezol Mechanosensors Optimize Human T Cell Activation. The Journal of Immunology January 12, 2018, ji1701118) disclose that mechanical forces contribute to optimal T cell activation, as reflected by the superior efficiency of immobilized TCR-cross-linking Abs compared with soluble Abs in TCR triggering, although a dedicated mechanotransduction module is not known. They found that the mechanosensor protein Piezo 1 is critically involved in human T cell activation, and provide the first evidence, to their knowledge, for the involvement of Piezol mechanosensors in immune regulation.

Ranade et al. (in: Ranade et al. Piezol, a mechanically activated ion channel, is required for vascular development in mice. PNAS. 2014 Jul 15;111(28):10347-52) identified Piezol and Piezo2 as mechanically activated cation channels that Piezol is expressed in endothelial cells of developing blood vessels in mice and highlight an essential role of mammalian Piezol in vascular development during embryonic development.

It would be highly beneficial to gain more insight into the factors that impact the organization of multiple ECs into freestanding tubular structures, in order to, for example, allow a better therapeutic use and control over angiogenic processes.

According to a first aspect of the present invention, this object is solved by an isolated mammalian CD11b+/PIEZO-1+ macrophage cell or neutrophil cell.

In another aspect of the present invention, the object is solved by a pharmaceutical composition comprising the CD11b+/PIEZO-1+ macrophage cell or neutrophil cell according to the present invention, together with a pharmaceutically acceptable carrier and/or auxiliary agent.

In yet another aspect of the present invention, the object is solved by a method for producing a pharmaceutical composition according to the present invention, comprising the steps of comprising the steps of a) providing a biological sample derived from a mammal comprising CD11b+ macrophages or neutrophil cells, b) purifying and/or isolating CD11b+ PIEZO-1+ macrophages or neutrophil cells from said sample based on the expression of CD11b+ and PIEZO-1+, and c) formulating said purified and/or isolated CD11b+ PIEZO-1+ macrophages or neutrophil cells into a suitable pharmaceutical composition.

In yet another aspect of the present invention, the object is solved by a method for diagnosing angiogenesis in a mammal, comprising identifying CD11b+ PIEZO-1+ macrophages or neutrophil cells, in a sample from a mammal to be diagnosed, wherein the presence of CD11b+ PIEZO-1+ macrophages or neutrophil cells is indicative for angiogenesis in said mammal, when compared to a control sample.

In yet another aspect of the present invention, the object is solved by a method for identifying a compound that modulates the expression and/or the biological activity of PIEZO-1 in a CD11b+ macrophage or neutrophil cell, comprising the steps of a) contacting said CD11b+ macrophage or neutrophil cell with at least one compound that potentially modulates the expression and/or the biological activity of PIEZO-1 in said CD11b+ macrophage or neutrophil cell, and b) identifying a modulation of the expression and/or the biological activity of PIEZO-1 in the presence of said at least one compound, compared to a control CD11b+ macrophage or neutrophil cell.

Vascularization is a critical step in the restoration of cellular homeostasis. Several strategies including localized growth factor delivery, endothelial progenitor cells, genetically engineered cells, gene therapy, and pre-vascularized implants have been explored to promote re-vascularization. But, long-term stabilization of newly-induced vessels remains a challenge. It has been shown that fibroblasts and mesenchymal stem cells can stabilize newly-induced vessels. However, whether an injected biomaterial alone can serve as an instructive environment for angiogenesis remained to be elucidated.

The present invention is based on the surprising finding that appropriate vascular branching, and long-term stabilization can be promoted simply by implanting a hydrogel with stiffness matching that of fibrin clot. During experiments in the context of the present invention in order to identify an underlying physiological mechanism, the inventors surprisingly identified a new and unique sub-population of circulating CD1 1b+ myeloid cells that express the stretch activated cation channel Piezo-1 which is recruited in the muscle-like hydrogel and in the fibrin clot-like hydrogel. These findings offered also evidence for a mechanobiology paradigm in angiogenesis involving an interplay between mechano-sensitive circulating cells and mechanics of tissue microenvironment.

The present invention thus employs the above findings in the context of conditions related to angiogenesis in several ways.

First, a yet unknown method for diagnosing angiogenesis in a mammal can be established, where CD1 1b+ PIEZO-1+ macrophages and/or neutrophil cells in a sample from a mammal to be diagnosed are suitably identified. The presence of these CD11b+ PIEZO-1+ macrophages and/or neutrophil cells is then indicative for angiogenesis in said mammal, when compared to a control sample. Said identifying can further comprise detecting the amount of said CD11b+ PIEZO-1+ macrophages, neutrophil cells and/or the level of expression and/or the levels of the biological activity of CD11b+ and PIEZO-1+ in said macrophages and/or neutrophil cells in said sample. Consequently, an increase of the amount of said CD11b+ PIEZO-1+ macrophages and/or neutrophil cells and/or the level of expression and/or the levels of the biological activity of CD11b+ and PIEZO-1+ in said macrophages and/or neutrophil cells, when compared to a control, is indicative for an increased angiogenesis.

Methods to detect the amount of said CD11b+ PIEZO-1+ macrophages and/or neutrophil cells and/or the level of expression and/or the levels of the biological activity of CD11b+ and PIEZO-1+ in said macrophages and/or neutrophil cells in said sample are known to the person of skill, and may comprises the use of beads, such as Dynal^{®} beads or cell sorting, such as fluorescence activated cell sorting (FACS^{®}). Furthermore, methods based on marker specific antibodies (CD11b+ and/or PIEZO-1+), or respective quantitative PCR, such as rtPCR, are known, and can be used.

Second, since the mammalian CD11b+/PIEZO-1+ macrophage cell and/or neutrophil cell according to the present invention are relevant for angiogenesis, the cells themselves provide valuable pharmaceutical compounds and components. Hence, the present invention provides such components, preferably as a pharmaceutical composition comprising the CD1 1b+/PIEZO-1+ macrophage cell and/or neutrophil cell according to the present invention, together with a pharmaceutically acceptable carrier and/or auxiliary agent.

In particularly preferred embodiments of the pharmaceutical composition according to the present invention said pharmaceutical composition further advantageously comprises a matrix material selected from the group of alginate, hyaluronic acid, agarose, poloxamers (e.g. Pluronic^{®}), polyethylene (PEG) hydrogels, and a modified primary hydroxyl groups containing polysaccharide comprising repeating disaccharide units, such as, for example, agarose, wherein in at least part of the disaccharide units the primary hydroxyl group is replaced by functional groups selected from halide groups or groups comprising sulfur or phosphorus atoms, such as, for example, sulfate groups, sulfonate groups, phosphonate groups and phosphate groups, preferably carboxylated agarose and phosphate agarose. This biomaterial can serve as an (additional) instructive environment for angiogenesis.

Generally, such a pharmaceutical composition according the present invention can be produced following the steps of a) providing a biological sample derived from a mammal comprising CD11b+ macrophage cells and/or neutrophil cells
, b) purifying and/or isolating CD11b+ PIEZO-1+ macrophage cells and/or neutrophil cells from said sample based on the expression of CD1 1b+ and PIEZO-1+, and
c) formulating said purified and/or isolated CD11b+ PIEZO-1+ macrophage cells and/or neutrophil cells into a suitable pharmaceutical composition. Preferably, said purifying and/or isolating comprises the use of beads, such as Dynal^{®} beads or cell sorting, such as fluorescence activated cell sorting (FACS^{®}), e.g. based on suitable cellular markers as described herein.

Nevertheless, the present invention also relates to several other useful pharmaceutical compositions and related methods of making these. In one aspect, these pharmaceutical compositions comprise a compound that modulates the expression and/or the biological activity of PIEZO-1 in a CD11b+ macrophage or neutrophil cell (or precursors thereof). Preferably, said modulation is an induction and/or increase of said expression and/or biological activity of PIEZO-1 in said macrophage or neutrophil cell (or precursors thereof).

This compound can be used in an in vitro method for producing a CD11b+/PIEZO-1+ macrophage or neutrophil cell, comprising the steps of a) providing a biological sample, in particular blood, comprising CD1 1b+ macrophages or precursor monocytes, and b) in vitro differentiating said CD11b+ macrophages or precursor monocytes into CD11b+ PIEZO-1+ macrophages or neutrophil cells using suitable signal compounds, such as, for example, small molecules, cytokines, proteins, or peptides. Since CD11b+/PIEZO-1+ macrophages or neutrophil cells present a new group of yet unknown cells, not all compounds are known, and an inventive method for identifying a compound that modulates the expression and/or the biological activity of PIEZO-1 in a CD11b+ macrophage or neutrophil cell can be used that comprises the steps of a) contacting said CD11b+ macrophage or neutrophil cell with at least one compound that potentially modulates the expression and/or the biological activity of PIEZO-1 in said CD11b+ macrophage or neutrophil cell, and b) identifying a modulation of the expression and/or the biological activity of PIEZO-1 in the presence of said at least one compound, compared to a control CD11b+ macrophage. Preferably said compound is selected from the group consisting of a peptide library, a combinatory library, a cell extract, in particular a plant cell extract, a "small molecular drug", an antisense oligonucleotide, an siRNA, and an mRNA. Performing such screening methods is well-known in the art, for example a "contacting" in the present invention means any interaction between the potentially binding substance(s) with the CD11b+ macrophage or a precursor cell, whereby any of the two components can be independently of each other in a liquid phase, for example in solution, or in suspension or can be bound to a solid phase, for example, in the form of an essentially planar surface or in the form of part isles, pearls or the like. In a preferred embodiment a multitude of different potentially binding substances are immobilized on a solid surface like, for example, on a compound library chip and the CD11b+ macrophage or neutrophil cell or a precursor cell thereof is subsequently contacted with such a chip.

Measuring of binding of the compound to the CD1 1b+ macrophage, neutrophil cell or a precursor cell can be carried out either by measuring a marker that can be attached either to the cell (or is a marker for said cell) or to the potentially interacting compound. Suitable markers are known to someone of skill in the art and comprise, for example, fluorescence or radioactive markers. As a further step after measuring the binding of a potentially interacting compound and after having measured at least two different potentially interacting compounds at least one compound can be selected, for example, on grounds of the measured binding activity or on grounds of the detected increase or decrease of binding activity and/or expression.

The thus selected binding compound is then in a preferred embodiment modified in a further step. Modification can be effected by a variety of methods known in the art, which include without limitation the introduction of novel side chains or the exchange of functional groups like, for example, introduction of halogens, in particular F, Cl or Br, the introduction of lower alkyl groups, preferably having one to five carbon atoms like, for example, methyl, ethyl, n-propyl, isopropyl, n-butyl, isobutyl, tert-butyl, n-pentyl or iso-pentyl groups, lower alkenyl groups, preferably having two to five carbon atoms, lower alkynyl groups, preferably having two to five carbon atoms or through the introduction of, for example, a group selected from the group consisting of NH₂, NO₂, OH, SH, NH, CN, aryl, heteroaryl, COH or COOH group.

In another aspect, the pharmaceutical compositions are produced by a method comprising in vitro differentiating of said CD11b+ macrophages, neutrophil cells, or precursor monocytes into CD11b+ PIEZO-1+ macrophages or neutrophil cells, comprising the steps of a) providing a biological sample derived from a mammal comprising CD11b+ macrophages, neutrophil cells or precursor monocytes thereof, b) in vitro differentiating said CD11b+ macrophages, neutrophil cells or precursor monocytes into CD11b+ PIEZO-1+ macrophages or neutrophil cells, and c) formulating said purified and/or isolated CD11b+ PIEZO-1+ macrophages or neutrophil cells into a suitable pharmaceutical composition. Preferably, said purifying and/or isolating comprises the use of beads, such as Dynal^{®} beads or cell sorting, such as fluorescence activated cell sorting (FACS^{®}), e.g. based on suitable cellular markers as described herein.

In another aspect, the method according to the present invention further comprises a suitable in vitro expansion of the CD11b+ PIEZO-1+ macrophages or neutrophil cells before formulating into a pharmaceutical composition.

In general, disclosed is a method according to the present invention for manufacturing a pharmaceutical composition, comprising the steps of performing a method according to the present invention as above, and formulating said compound as identified into a pharmaceutical composition. The pharmaceutical compositions of the invention are preferably sterile and contain an effective amount of the agents described herein and optionally of further agents as discussed herein to generate the desired reaction or the desired effect, e.g. a pharmaceutical angiogenetic effect or a differentiation of said CD11b+ macrophages or neutrophil cells or precursor monocytes into CD11b+ PIEZO-1+ macrophages or neutrophil cells. Pharmaceutical compositions are usually provided in a uniform dosage form and may be prepared in a manner known per se. A pharmaceutical composition may e.g. be in the form of a solution or suspension. A pharmaceutical composition may comprise salts, buffer substances, preservatives, carriers, diluents and/or excipients all of which are preferably pharmaceutically acceptable. The term "pharmaceutically acceptable" refers to the non-toxicity of a material which does not interact with the action of the active component of the pharmaceutical composition. Salts which are not pharmaceutically acceptable may be used for preparing pharmaceutically acceptable salts and are included in the invention. Pharmaceutically acceptable salts of this kind comprise in a non-limiting way those prepared from the following acids: hydrochloric, hydrobromic, sulfuric, nitric, phosphoric, maleic, acetic, salicylic, citric, formic, malonic, succinic acids, and the like. Pharmaceutically acceptable salts may also be prepared as alkali metal salts or alkaline earth metal salts, such as sodium salts, potassium salts or calcium salts.

Suitable buffer substances for use in a pharmaceutical composition include acetic acid in a salt, citric acid in a salt, boric acid in a salt and phosphoric acid in a salt. Suitable preservatives for use in a pharmaceutical composition include benzalkonium chloride, chlorobutanol, paraben and thimerosal. An injectable formulation may comprise a pharmaceutically acceptable excipient such as Ringer Lactate.

The term "carrier" refers to an organic or inorganic component, of a natural or synthetic nature, in which the active component is combined in order to facilitate, enhance or enable application. According to the invention, the term "carrier" also includes one or more compatible solid or liquid fillers, diluents or encapsulating substances, which are suitable for administration to a patient. Possible carrier substances for parenteral administration are e.g. sterile water. Ringer, Ringer lactate, sterile sodium chloride solution, polyalkylene glycols, hydrogenated naphthalenes and, in particular, biocompatible lactide polymers, lactide/glycolide copolymers or polyoxyethylene/polyoxy- propylene copolymers. The term "excipient" when used herein is intended to indicate all substances which may be present in a pharmaceutical composition and which are not active ingredients such as, e.g., carriers, binders, lubricants, thickeners, surface active agents, preservatives, emulsifiers, buffers, flavoring agents, or colorants. The agents and compositions described herein may be administered via any conventional route, such as by parenteral administration including by injection or infusion. Administration is preferably parenterally, e.g. intravenously, intra-arterially, subcutaneously, intra-dermally or intramuscularly. Compositions suitable for parenteral administration usually comprise a sterile aqueous or non-aqueous preparation of the active compound, which is preferably isotonic to the blood of the recipient. Examples of compatible carriers and solvents arc Ringer solution and isotonic sodium chloride solution. In addition, usually sterile, fixed oils are used as solution or suspension medium.

The agents and compositions described herein are administered in effective amounts. An "effective amount" refers to the amount which achieves a desired reaction or a desired effect alone or together with further doses. In the case of treatment of a particular disease or of a particular condition, the desired reaction preferably relates to inhibition of the course of the disease. This comprises slowing down the progress of the disease and, in particular, interrupting or reversing the progress of the disease, in the present context by influencing angiogenesis. The desired reaction in a treatment of a disease or of a condition may also be delay of the onset or a prevention of the onset of said disease or said condition.

An effective amount of an agent or composition described herein will depend on the condition to be treated, the severity of the disease, the individual parameters of the patient, including age, physiological condition, size and weight, the duration of treatment, the type of an accompanying therapy (if present), the specific route of administration and similar factors. Accordingly, the doses administered of the agents described herein may depend on several of such parameters. In the case that a reaction in a patient is insufficient with an initial dose, higher doses (or effectively higher doses achieved by a different, more localized route of administration) may be used. The agents and compositions described herein can be administered to patients, e.g., in vivo, to treat or prevent a variety of disorders such as those described herein. Preferred patients include human patients having disorders that can be corrected or ameliorated by administering the agents and compositions described herein having a desired effect on angiogenesis.

Preferably, said pharmaceutical composition according to the present invention can be used for the in vitro production/induction of CD11b+ PIEZO-1+ macrophages or neutrophil cells as described herein.

Further preferably, said pharmaceutical composition according to the present invention is for administration to a patient in need thereof, and wherein said CD11b+ macrophages, neutrophil cells or precursor monocytes thereof are autologous, heterologous, such as xenogeneic, to said patient.

During angiogenesis, ECs express the αv family of integrins, which are transmembrane proteins that specifically bind to the arginine-glycine-aspartic acid (RGD) sequence found in many ECM molecules, including collagen, fibronectin, and vitronectin (9) Since integrins are also anchored to the actin cytoskeleton of the cell (10), they function as mechanotransducers and assist the cells in perceiving the mechanics of the ECM; and it has been shown that integrin signaling is necessary for both EC survival and proliferation. In spite of this compelling evidence linking mechanical cues to EC function and, the impact of the ECM mechanical properties on blood vessel sprouting and maturation remains unknown. The inventors had recently demonstrated *in vitro* that ECs when presented with a hydrogel environment of defined stiffness in combination with RGD-signaling and soluble pro-angiogenic signals can undergo apical-basal polarization and organize into free standing multicellular lumens even in absence of mural support cells. Encouraged by this observation in this study the inventors inquired if the mere introduction of such biomaterials with defined mechanical properties could be sufficient to promote maturation and stabilization of neovasculature. Specifically, the inventors chose a 2 wt-% solution of carboxylated agarose (CA) of 28% and 60% carboxylation as they yielded injectable gels representing two distinct stiffness (shear moduli) spanning one order of magnitude (5 kPa and 0.5 kPa, respectively) that mimic the mechanical properties of muscle (1.5 - 3.5 kPa) (13), and fibrin network on blot clot (0.06 - 0.6 kPa) (14), respectively; and additionally, can be formed *in situ* at physiological acceptable temperatures. To ensure mechanical coupling of ECs with the gel and to exploit the known benefits of RGD signaling maintenance of EC function, the CA was functionalized with peptide presenting the GGGGRGDSP sequence in the N-terminus using aqueous 1-Ethyl-3-(3-dimethylaminopropyl)carbodiimide (EDC) chemistry. Since RGD ligand density is known to impact cell-biomaterial interaction the reaction conditions were optimized to ensure an optimal density.

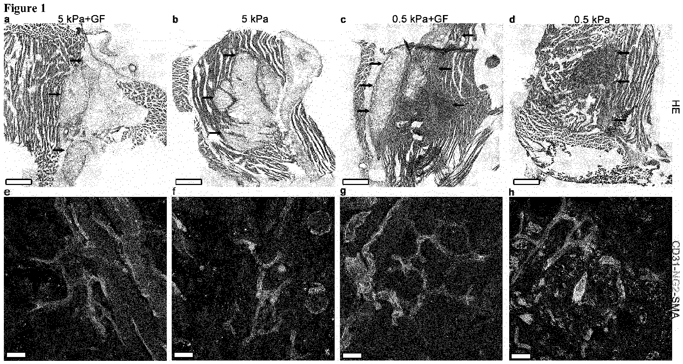

CA hydrogels of 5 kPa and 0.5 kPa stiffness, with or without soluble growth factor supplementation (GF) (four conditions), were implanted into gastrocnemius muscles, a target tissue relevant for periphery artery diseases, of SCID mice in order to evaluate the potential angiogenic response. Hematoxylin and Eosin staining showed that after 2 weeks, hydrogels were clearly evident in all conditions and well tolerated by the muscle tissue (Figure 1a-d).

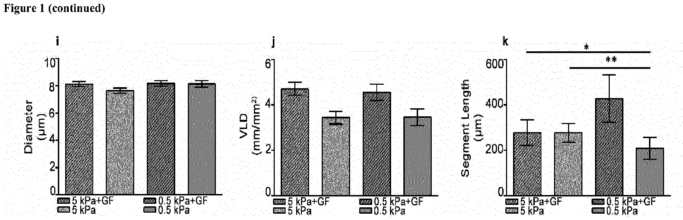

Since CA have a unique property profile such as tunable mechanical properties and *in situ* gelation (12), the observed tissue compatibility bodes well for the further utilization of CA in investigating mechanobiology paradigms. The ability of the CA gels to stimulate the ingrowth of blood micro-vessels into its avascular environment was investigated by immunofluorescence and confocal microscopy. All compositions were efficiently invaded by newly formed micro-vessels, physiologically associated with mural cells, i.e. pericytes (positive for nerve/glial antigen 2 (NG2) and negative for a-SMA), and smooth muscle cells (positive for a-SMA) (Figure 1e-h). All compositions induced similar degree of angiogenesis, as measured by vessel length density (VLD), i.e. the total length of vessels in a given area within the gel independently of their diameter, although the presence of GF slightly increased the number of capillaries inside the stiffer hydrogels (5kPa+GF = 4.7±0.3 vs 5kPa = 3.4±0.3 mm/mm2, *p < 0.05 and 0.5kPa+GF = 4.6±0.3 vs 0.5kPa = 3.5±0.4 mm/mm2, p = n.s.; Figure 1i). On the other hand, functionality of micro-vascular networks correlates with a high degree of branching (short segment length) (15) and a moderate diameter in the range of capillaries (5-10 µm). Interestingly, GF supplementation did not improve the quality of the induced vascular networks (Figure 1j-k). In fact, the shortest segment length was actually achieved by the softer 0.5 kPa gel alone (5kPa+GF = 278.5±55.6 µm vs 5kPa = 277.1±41.3 µm, p = n.s.; and 0.5kPa+GF = 428.5±103.6 µm vs 0.5 kPa = 207.8±48.2 µm, p=n.s.) and vessel diameters were similar in all conditions (5kPa+GF = 8.1±0.2 µm vs 5kPa = 7.7±0.2 µm, p=n.s.; and 0.5kPa+GF = 8.2±0.2 µm vs 0.5 kPa = 8.2±0.2 µm, p=n.s.). Taken together, these data show that both soft and stiff hydrogels were similarly effective in inducing initial vascular ingrowth and that the addition of growth factors does not confer any significant advantage.

Matrigel - a matrix derived from Engelbreth-Holm-Swarm (EHS) mouse sarcoma cells rich in basal lamina proteins and growth factors (16) - has been shown to be highly supportive of vascular growth, and is therefore widely used to stimulate angiogenesis both *in vitro* and *in vivo.* In order to further improve the supportive properties of the hydrogel for vascular ingrowth, the different compositions were supplemented with Matrigel (0.01 % w/v) and the outcomes were ascertained after two weeks implantation in muscle.

It is important to note that the addition of Matrigel has no impact on the mechanical properties of the gel as shown earlier. All hydrogels were well integrated in the muscle tissue, similar to the unsupplemented conditions. The amount of micro-vascular ingrowth induced by the addition of Matrigel was similar to stiffness environments with GF (5kPa+Mat = 4.4±0.4 and 0.5kPa+Mat = 4.8±0.3 mm/mm²) although slightly increased compared to vascular density induced by hydrogels alone (p = n.s. vs 5kPa and 0.5kPa). However, Matrigel addition caused capillary networks that were much less branched, as evidenced by the significantly longer average segment length (5kPa+Mat = 671.4±118.5 µm, vs 5kPa = 277.1±41.5; 0.5kPa+Mat= 1115.0±140.3 µm, vs 0.5kPa = 207.8±48.2 µm and p<0.0001), suggesting a less beneficial connectivity compared to both hydrogel conditions. Furthermore, in the presence of Matrigel, vascular morphology was still that of normal capillaries but with a moderately smaller caliber compared to the unsupplemented hydrogel alone conditions (5kPa+Mat = 6.4±0.2 µm vs 5kPa = 7.7±0.2 µm, **** p<0.0001 and 0.5kPa+Mat = 6.0±0.2 vs 0.5kPa = 8.2±0.2 µm, **** p<0.0001). Overall, these data show that Matrigel supplementation did not improve the early pro-angiogenic properties of the hydrogels alone.

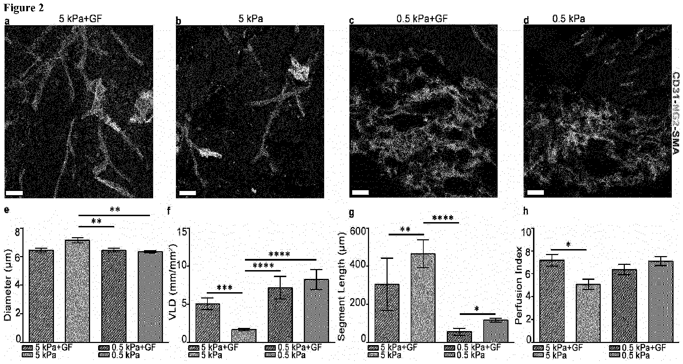

In order to be therapeutically useful, newly induced vascular structures must stabilize, i.e. persist long-term without regression. Newly induced vessels require about 4 weeks to become independent of further angiogenic stimuli and persist indefinitely (19). Therefore, the fate of initially induced vascular structures was investigated 7 weeks after implantation of 5 and 0.5kPa hydrogels with and without GF in murine hind limb muscles. Since GF and Matrigel supplementation yielded similar outcomes, the condition with GF alone was included in this experiment in order to account for their role during initial vessel induction. Masson trichrome staining revealed that compared to the 2-week 125 time point, after 7 weeks hydrogels continue to persist at the site of implantation, supported efficient infiltration of host cells, and induced no foreign-body reaction as assessed by the absence of a collagenous capsule, suggesting that the gels were integrated in muscle tissue. Quantification of the vasculature showed a significant regression of the capillaries initially induced within the 5kPa hydrogels, as VLD was reduced by >50% compared to the 2-week time-point from 4.4±0.4 to 1.7±0.2 mm/mm² (p<0.0001), although GF supplementation allowed a better stabilization (VLD 7wk = 5.0±0.8 mm/mm² vs 2wk = 4.7±0.3 mm/mm², p = n.s.) (Figure 2a, 2b, 2e). In contrast, the softer 0.5kPa hydrogels not only prevented any regression, but actually promoted further network expansion (VLD 7wk = 8.1±1.3 mm/mm2 vs 2wk = 3.6±0.3 mm/mm², p<0.0001) regardless of GF supplementation (0.5kPa+GF = 7.2±1.5 mm/mm², p = n.s. vs 0.5kPa), yielding significantly denser vascularity than the harder 5kPa hydrogel (Figure 2c, 2d, 2e). While in the 5kPa hydrogels vessel regression was accompanied by a reduction in network branching, with a 40% increase in vascular segment length from 277.1±41.3 µm to 465.2±73.7 µm (p = n.s.), capillary networks in the softer 0.5kPa gels further increased their branching degree compared to the 2-week time-point, again regardless of GF supplementation (segment length 0.5kPa = 117.4±10.5 µm and 0.5kPa+GF = 55.3±18.5 µm). Furthermore, while vessels in the 5kPa hydrogels were scarcely associated with mural cells of any kind (Figure 2a-b), networks in the 0.5kPa gels were associated with NG2+ pericytes, like normal muscle capillaries (Figure 2c-d) which have been shown to play a role in vessel stabilization. Vessel diameters were similar among all groups and comprised in the 5-10 µm capillary range (Figure 2g). Perfusion has been shown to promote the stabilization of nascent vascular structures.

Therefore, the establishment of functional blood flow in newly induced vascular structures was assessed by intravenous injection of biotinylated tomato lectin 150 that binds the luminal surface of blood vessels and marks only vessels that are functionally perfused by the systemic circulation. Quantification of lectin perfusion showed that vessels in all conditions were well perfused (about 70% of lectin+ endothelial structures), with only the 5kPa gels showing a moderate reduction to about 50% (Figure 2h). This provides evidence the RGD-modified CA supports formation of fully functional vascular networks.

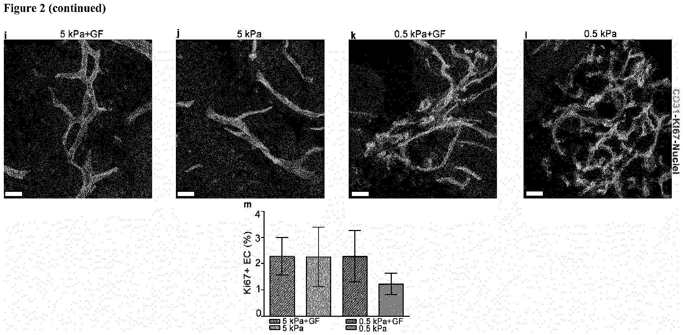

Since the 0.5kPa gels at 7 weeks showed a moderate increase in the amount of newly induced capillaries compared to 2-week time-point, endothelial proliferation was investigated by immunostaining for Ki67, which marks the nucleus of cells in all phases of the cell cycle (G1, S, G2, and M), excluding quiescent ones (G0) (22). Quantification of Ki67+ endothelial nuclei showed that vascular networks in all gel compositions were essentially quiescent, with at least 98% of ECs in G0 phase (Figure 2i-m). This is consistent with previous findings that in fully normal angiogenesis induced by VEGF 93% of ECs are already in G0 phase after 1 week. Taken together, these data suggest that: a) softer 0.5kPa hydrogel specifically promotes new vessel stabilization, yielding long-term persistent and mature (pericytes associated) micro-vascular networks with the most optimal functional features of high density and branching complexity, and b) GF supplementation does not improve the long-term angiogenic effect that are already imposed by the mechanical environment of the hydrogels.

Circulating myeloid cells can be recruited to sites of active angiogenesis and play a role in both maturation and stabilization of new vessels (24). In particular, a specific population of CD11b+ monocytes called Neuropilin-Expressing Monocytes (NEM), as they express Neuropilin-1 (Nrp1), a co-receptor for VEGF and Semaphorin3A co-receptor, has been recently found to accelerate new vessel stabilization, both directly by activating TGF-blsignaling and indirectly by promoting pericyte recruitment through PDGF-BB secretion.

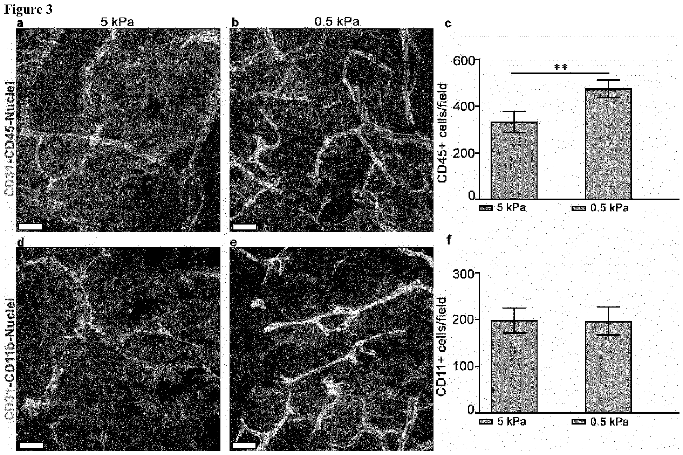

Therefore, the inventors investigated whether the long-term stabilization of the capillary networks by the 0.5kPa hydrogel could relate to a differential recruitment of myeloid cells and specifically pro-maturative CD11b+ monocytes. Two weeks after implantation into hind limb muscles, immuno fluorescent staining showed that the 0.5kPa hydrogels recruited about 40% more CD45+ myeloid cells than the harder 5kPa composition (476.9±37.7 vs 333.8±44.7 cells/field, p<0.01; Figure 3a-c). On the other hand, the recruitment of CD11b+ cells were similar in both gel compositions (Figure 3d-f).

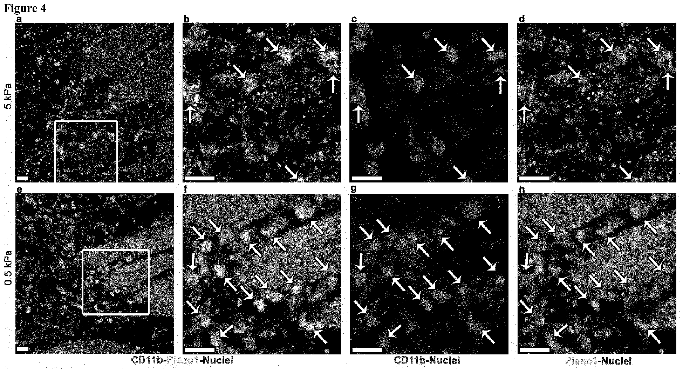

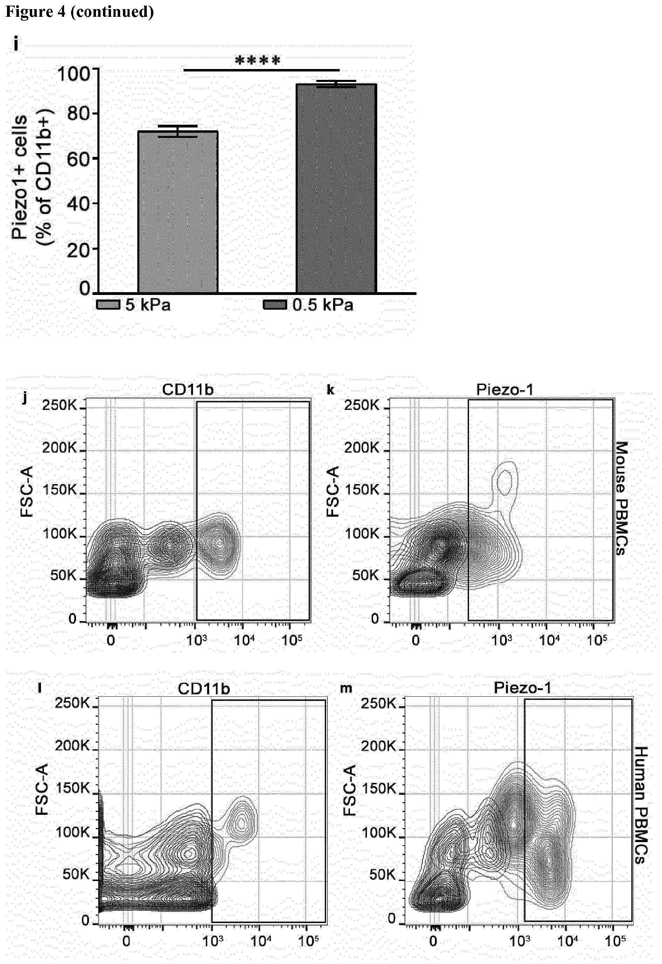

Since, mechanical stiffness was the only difference between the hydrogel promoting vessel stabilization and persistence (softer, 0.5 kPa) and the ineffective one (harder, 5 kPa), the inventors inquired if there exists a mechano-sensitive cell population that is preferentially recruited to the softer hydrogel microenvironment. Immunofluorescence staining identified for the first time a hitherto unknown population of CD11b+ monocytes expressing Piezo-1 (Figure 4a-h), which was significantly more frequently found in the softer than in the harder hydrogels, representing 93.1±1.4% of the total CD11b+ monocytes in the 0.5kPa gels 200 vs 71.8±2.4% in the 5kPa ones (p<0.0001; Figure 4i). In order to ascertain if a Piezo-1+ monocytes exist in circulation and are recruited into the gels, or Piezo-1 expression is induced in CD11b+ monocytes upon exposure to the gel microenvironment, both mouse and human peripheral blood mononuclear cells were analyzed using flow cytometry. A population of Piezo-1-expressing CD11b+ monocytes were identified in the circulation of both mouse and healthy human donors (Figure 4j-n).

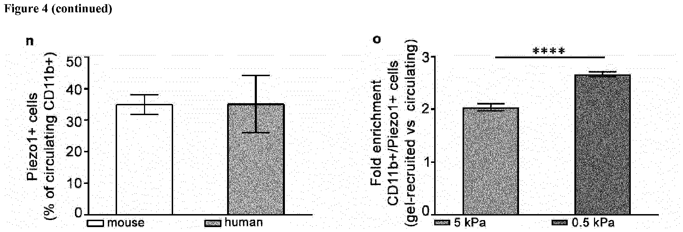

Interestingly, the frequency of this population of mechano-sensitive monocytes was similar in mouse and human circulation, accounting for 35.0±2.2% and 35.1±9.1% of total CD11b+ monocytes respectively (p = n.s.; Figure 4n). Interestingly, this mechano-sensitive monocyte population was specifically enriched within the implanted hydrogels compared to its frequency in the circulation. Further, the softer gels were 35% more effective in recruiting CD 11 b+/Piezo 1+ monocytes than the harder gels (5kPa=2-fold enrichment compared to the frequency in the circulation vs 0.5kPa=2.7-fold, p<0.0001; Figure 4o). Therefore, these data suggest that a population of mechano-sensitive CD 11 b+/Piezo 1 + monocytes exist in normal circulation and that they can be preferentially recruited to CA hydrogels in differential manner based on their mechanical properties. In particular, considering the recently identified functions of CD11b+ monocytes in regulating the stabilization of newly induced vessels, this novel population may represent the link between the mechanics and angiogenic properties of hydrogels and represents a novel direction for future effort in developing systems and pharmacological agents for therapeutic angiogenesis.

Vascularization is a critical step in the restoration of cellular homeostasis. Several strategies including localized growth factor delivery, endothelial progenitor cells, genetically engineered cells, gene therapy, and pre-vascularized implants have been explored to promote re-vascularization. But, long-term stabilization of newly-induced vessels remains a challenge. It has been shown that fibroblasts and mesenchymal stem cells can stabilize newly-induced vessels. However, whether an injected biomaterial alone can serve as an instructive environment for angiogenesis remains to be elucidated. The inventors found that appropriate vascular branching, and long-term stabilization can be promoted simply by implanting a hydrogel with stiffness matching that of fibrin clot. They have furthermore identified a unique sub-population of circulating CD11b⁺ myeloid cells that express the stretch activated cation channel Piezo-1 which is enriched prominently in the clot-like hydrogel. These findings offer evidence for a mechanobiology paradigm in angiogenesis involving an interplay between mechano-sensitive circulating cells and mechanics of tissue microenvironment.

The present invention is described in detail by the figures and examples below, which are used only for illustration purposes and are not meant to be limiting.
Figure 1 shows that RGD functionalized carboxylated agarose hydrogels induce angiogenesis. a-d Frozen sections of GC muscles implanted with distinct hydrogel compositions were stained for hematoxylin/eosin and (e-h) immunostained against CD31 (endothelial cells, red), NG2 (pericytes, green), α-SMA (smooth muscle cells, cyan). Quantification of vessel morphology: Vessel diameters (i) and vascular segment length (j) were quantified in the same areas within the hydrogels two weeks post implantation: VLD = vessel length density, is expressed as millimeters of vessel length per square millimeter of area of effect (mm/mm²); the segment length is expressed as µm of vessel length between 2 consecutive branch points (k). All data sets represent mean values ± SEM; * p<0.05, ** p<0.01 by Kruskal-Wallis test; n= 4 independent muscles per each group. Scale bars = 1 mm in all HE-stained panels. Scale bars = 20 µm in all immunofluorescence-stained panels.
Figure 2 shows that soft RGD-functionalized carboxylated agarose supports stable capillaries, a-d Immunofluorescence staining of endothelium (CD31, in light gray), pericytes (NG2, in gray), smooth muscle cell (α-SMA, in dark gray) on frozen sections of leg skeletal muscles of mice injected with distinct hydrogel compositions and sacrificed 7 weeks later. e-h Seven weeks post hydrogel implantation the VLD, the vascular segment length, vessel diameters and perfusion index were quantified in the same areas within the hydrogels: All data sets represent mean values ± SEM; * p<0.05, ;** p<0.01, *** p<0.001 and ****p<0.0001 by Kruskal-Wallis test; n= 4 independent muscles per each group. i-m Endothelial proliferation was assessed by quantifying the percentage of endothelial cells positive for Ki67 by immunofluorescence staining on frozen muscle sections, n= 4 independent muscles per group. Scale bars = 20 µm in all immunofluorescence-stained panels.
Figure 3 shows that RGD functionalized carboxylated agarose recruits myeloid cells, a-f Immunofluorescence staining of endothelial cells (CD31, in green), leukocyte (CD45, in red) and monocytes (CD11b, in green) on cryosections of limb muscles 2 week after injection with 5kPa and 0.5 kPa hydrogel compositions. Scale bar= 20µm in all panels. c, f Quantification of the number of CD45+ and CD11b+ cells recruited into the implanted hydrogel. All data sets represent mean values ± SEM;** p<0.01, by Mann-Whitney test; n= 4 independent muscles per each group.
Figure 4 shows that the soft RGD functionalized carboxylated agarose microenvironment recruits CD11b⁺/Piezo-1⁺ cells, (a-h) Immunofluorescence staining of monocytes (CD11b, in red) and of PIEZO-1⁺ cells (in light blue) on cryosections of limb muscles 2 week after injection with 5kPa and 0.5 kPa hydrogel compositions. Scale bar= 20µm in all panels. (i) Quantification of the number of CD11b⁺/Piezo1⁺ cells in sites within the implanted gels. (j-n) Circulating Piezo-1⁺/CD11b⁺ monocytes were identified in both mouse and human blood by FACS. (o) Fold enrichment of recruited CD11b⁺/Piezo1⁺ monocytes in sites within the implanted gels compared with circulating CD11b⁺/Piezo1⁺ monocytes. All data sets represent mean values ± SEM; **** p<0.0001, by parametric unpaired t-test; n= 3 mice and n= 3 human donors.

### EXAMPLES

### Materials ad Methods

### Gels preparation and characterization

Native agarose (1 g) (Merck, Darmstadt, Germany) was transferred into a 3-necked round bottom flask equipped with a mechanical stirrer and pH-meter (WTW, Weilheim, Germany), and dissolved in deionized water at a concentration of 1% w/v by heating to 90 °C. The flask was cooled down to 0°C, using an ice bath, under vigorous mechanical stirring in order to prevent gelation of the agarose, and the reactor was charged with 99% (2,2,6,6-tetramethylpiperidin-1-yl)oxyl (TEMPO) (20.6 mg, 0.16 mmol), NaBr (0.1 g, 0.9 mmol), and NaOCl (2.5 mL, 15% solution) all obtained from Sigma Aldrich (Steinheim, Germany). As the reaction occurs, the solution becomes acidic. The pH of the solution was maintained at 10.8 by dropwise addition of NaOH (0.1M) (Sigma-Aldrich Chemie GmbH, Steinheim, Germany) throughout the duration of the reaction. The degree of carboxylation was back calculated by using the volumes of NaOH (0.1 M)solution added during the reaction. The reaction was quenched by the addition of NaBH4 (0.1 g) (Sigma-Aldrich Chemie GmbH, Steinheim, Germany), following which the solution was acidified to pH 8 (0.1mHCl) and stirred for 1 h. The modified agarose was then precipitated by the sequential addition of NaCl (12 g, 0.2 mol) and ethanol (500 mL) (technical grade). The product was collected by vacuum filtration using a fritted glass funnel and then washed using ethanol (500 mL). The ethanol, catalyst, and salts were removed by extensive dialysis against water for 2 days with replacement of the water every 12 h. The modified agarose was then freeze-dried on a Beta 2-8 LD (Martin Christ Gefriertrocknungsanlagen GmbH, Osterode am Harz, Germany) overnight to yield a white solid. The degree of carboxylation was verified by the appearance of peaks associated with aliphatic carboxylic acid groups via FTIR (KBr) (vc=o: 1750 cm-1) (Bruker Optics, Ettlingen, Germany) and NMR 300 Mhz (13C: 180 ppm) (Bruker BioSpin, Rheinstetten, Germany).

### Carboxylated agarose mechanical properties

Rheology experiments were performed with a Physica MCR 301 (Anton Paar, Wundschuh, Austria) equipped with a Peltier cell to control the temperature and the experiment was performed with a plate geometry PPR25 (Anton Paar, 250 Wundschuh, Austria). Samples in deionized water were prepared by heating at 90 °C for 10 min until a clear solution was obtained. The liquid was then poured on the rheometer plate and the following sequence was used to determine the shear modulus: cool down from 80 °C to 5 °C in 30 min, 30 min equilibration at 5 °C to allow the gel to form, followed by heating to 37 °C and equilibration for 30 min prior to measuring G' and G" by increasing the rotation frequency from 0.01 rad/s up to 10 rad/s with a 1% deformation. The G' of the gel was determined at 1 Hz shear frequency.

### Carboxylated agarose RGD functionalization

Functionalization of CA with the RGDSP (Peptides International, Louisville, Kentucky, USA) peptide was performed using 1-ethyl-3-(3-dimethyl- aminopropyl)carbodiimide (EDC) (Sigma-Aldrich Chemie GmbH, Steinheim, Germany) coupling chemistry. CA was sterilized overnight in 70% ethanol, and the ethanol was re-moved by extensive dialysis against water.

The sterile CA was freeze-dried overnight to yield a white solid. CA (30 mg, 0.25 µmol) was dissolved in MES sterile buffer (Sigma-Aldrich Chemie GmbH, Steinheim, Germany) and an excess EDC (210 mg, 1.3 mmol) was added and the solution stirred for 30 min. Following this the peptide was added (500 µg, 0.66 µmol for the CA-60 gels and 1 mg, 1.33 µmol for the gels) and the solution stirred for an additional 2 h at room temperature. Unreacted reagents were removed by dialysis against water. RGD incorporation was verified using elemental analysis Vario EL (Elementar Analysen systeme GmbH, Langenselbold, Germany) equipped with a thermal conductivity detector and an adsorption column for CO2 at 110 °C and H2O at 150 °C. All samples were accurately weighed to 3 mg before measurements and the percentage of nitrogen was used to calculate the peptide attachment. It was found that 11.6 ± 0.9 % of the repeat units where functionalized on both CA-28 and CA-60.

### Gels implantation in two

Gels were implanted into 10-15 week old immune-deficient SCID CB.17 mice (Charles River Laboratories, Sulzfeld, Germany). Animals were treated in accordance with Swiss Federal guidelines for animal welfare, and the study protocol was approved by the Veterinary Office of the Canton of Basel-Stadt (Basel, Switzerland; Permit 2071). Gels were pre-loaded in 1 ml syringes and kept on ice. Fifty µl of cold PBS (Sigma-Aldrich Chemie GmbH, Steinheim, Germany) followed by 50 µl of cold gels were implanted in both the *medialis* and *lateralis* portions of Gastrocnemius (GC) leg muscle, using a syringe with a 291/2G needle (Becton Dickinson, Allschwil, Switzerland).

### Tissue staining and microscopy

For the studies performed on frozen tissue sections, mice were anesthetized and the tissues were fixed by vascular perfusion of 1% paraformaldehyde in PBS pH 7.4 for 4 minutes under 120 mm/Hg of pressure. GC muscles were harvested, post fixed in 0.5% paraformaldehyde in PBS for 2 hours, cryo-protected in 30% sucrose in PBS overnight at 4°C, embedded in OCT compound (CellPath, Newtown, Powys, UK), frozen in freezing isopentane and cryosectioned. Tissue sections (30 µm) were stained with Hematoxylin and eosin (H&E) and in addition, the gel biocompatibility was examined with Masson trichrome staining (Réactifs RAL, Martillac, France), performed according to manufacturer's instructions. For immuno fluorescent staining of neighboring 30 µm thick longitudinal cryosections, the sections were blocked with PBS 0.1% triton supplemented with 5% normal goat or donkey serum and 2% BSA (all reagents from Sigma-Aldrich Chemie GmbH, Steinheim, Germany) for 1 hour. The slides were then incubated for 1.5 hour at room temperature with the following primary antibodies and dilutions: rat anti-mouse PECAM-1 (clone MEC 13.3, BD Biosciences, Basel, Switzerland) at 1:100 or hamster monoclonal 300 anti-mouse CD31 (clone 2H8, Millipore, Merck, Germany) at 1:200; mouse anti-mouse/human a-SMA (clone 1A4, MP Biomedicals, Basel, Switzerland) at 1:400; anti-mouse NG2 (Chemicon International, Hampshire, UK) at 1:200; rabbit anti-Ki67 (Abcam, Cambridge, UK) at 1:100; rat monoclonal anti-CD11b (clone M1/70, Abcam, Cambridge, UK) at 1:100; rat anti-mouse CD45 (PE conjugated, clone 30 F11, BD Biosciences, Basel, Switzerland) at 1:400. Negative controls lacking primary antibody were always performed. Sections were rinsed in PBS 0.1% triton and then incubated for 1.5 hour at room temperature with fluorescently labeled secondary antibodies (Invitrogen, Basel, Switzerland) diluted at 1:200. The slides were then rinsed and mounted.

Piezo1 staining immunohistochemistry experiments were performed on Ventana Discovery Ultra instrument (Roche Diagnostics, Manheim, Germany) by using the procedure RUO Discovery Universal instead. Cryosections were fixed for 12 minutes with 4% paraformaldehyde followed by 1 hour incubation at 37°C with rat anti-CD11b (1:100) and 32 minutes incubation at 37°C with a fluorescently labeled anti rat secondary antibody used at 1:200 (Invitrogen, Basel, Switzerland). Next, after an antibody denaturation step, sections were pre-treated for 16 minutes with Cell Conditioning Solution (CC1) (Roche Diagnostics, Mannheim). Rabbit anti Piezol (Proteintech, Manchester, UK) diluted at 1:500 was then incubated for 1hour at 37°C and detected with the secondary antibody (ImmPRESS reagent kit peroxidase anti-rabbit Ig MP-7401, Vector) applied manually (200 µl) for 32 minutes.

Discovery Rhodamine (Roche Diagnostics, Mannheim) applied for 12 minutes was used for the detection. To study vessel perfusion, 100 µg of biotinylated Lycopersicon esculentum (tomato) lectin (Vector Laboratories, Burlingame, California) was dissolved in 100 µl, which binds the luminal surface of all blood vessels, and injected intravenously through the femoral vein. After four minutes the thoracic cavity was opened and the tissues were fixed by perfusing the animal with 1% paraldehyde and leg muscle were collected and processed as described above. Fluorescently labeled Streptavidin (eBioscience, Vienna, Austria) at 1:200 was used to visualize the perfused vasculature. Frozen sections were mounted with Paramount Aqueous Mounting Medium (Dako, Agilent Technologies, Basel, Switzerland), and fluorescence images were taken with 40x objectives on a Carl Zeiss LSM710 3-laser scanning confocal microscope (Carl Zeiss, Feldbach, Switzerland) or with a 20X objective on an Olympus BX61 microscope (Olympus, Volketswil, Switzerland). All Image analysis were performed with either Cell Sense software (Olympus, Volketswil, Switzerland) or Imaris 7.6.5 software (Bitplane, Zürich, Switzerland) on fluorescence images acquired with a 20X objective on an Olympus BX61 microscope or with a 40x objective on a Carl Zeiss LSM710 3-laser scanning confocal microscope.

### Histological analysis

The quantification of vessel length density (VLD) and vessel perfusion was performed on sections of leg muscles harvested after intravascular staining with biotinylated lectin and fluorescently labeled streptavidin, as described above. After co-staining with a fluorescent anti-CD31 antibody, VLD was measured on 6-10 randomly acquired fields per leg and 4 muscles per group (n = 4) by tracing the total length of vessels in the fields and dividing it by the area of the fields. The total lengths of lectin-positive and CD31-positive vascular structures in each field were traced independently and the vessel perfusion index was calculated as the ratio between the two values. Vascular segment length was also measured in all representative fields per muscles tracing the total length of vessels and dividing it for the number + 1 of branching points. Vessel diameters were measured by overlaying a captured microscopic image with a square grid. Squares were chosen at random, and the diameter of each vessel (if any) in the center of selected squares was measured. Two to five hundred total vessel diameter measurements were obtained from 4 muscles 350 per each group (n = 4). K167+ endothelial cells (ECs) were quantified from the total number of ECs (260-890 total ECs were counted per condition at 7 weeks post gel implantation) in vascular structures visible in each of 3-5 fields, in each area of effect. 10 areas with a clear angiogenic effect were analyzed per group. The quantification of leukocytes (CD45) and monocyte (CD1 1b) were performed on 7 random areas per muscle (n = 4) per group by counting them and normalizing to the absolute number of CD45+ and CD11b+ cells with area. (9000-1400 total CD45+ cells and 5000-6000 total CD11+ cells were counted per condition at 2 weeks post gel implantation). The quantification of Piezo 1 +/CD 11 b+ cells was performed on 7-10 random areas per muscle (n = 4) after immunostaining for CD11b (700-800 total CD1 1+ cells were counted per condition at 2 weeks post gel implantation).

All Image measurements were performed with both Cell Sense software (Olympus Volketswil, Switzerland) and Imaris 7.6.5 software on fluorescence images acquired with a 20X objective on an Olympus BX61 microscope or with a 40x objective on a Carl Zeiss LSM710 3-laser scanning confocal microscope.

### Blood cell analysis by FACS

Peripheral Blood Mononuclear Cells (PBMCs) were isolated from 3 immune-deficient SCID CB.17 mice (Charles River Laboratories, Sulzfeld, Germany) and 3 human healthy donor using a density gradient technique (Histopaque-1077, Sigma-Aldrich Chemie GmbH, Steinheim, Germany). Briefly, after centrifugation, carefully aspirated PBMCs from the Ficoll-plasma interface were stained for APC-anti-human CD11b (clone ICRF44, BD Biosciences, Basel, Switzerland) at 1:100 or rat monoclonal anti-CD11b at 1:100 and anti-human Piezol (Abcam, Cambridge, UK) at 1:500. Fluorescently labeled secondary antibody to detect Piezo1 (Invitrogen, Basel, Switzerland) was used at 1:200. Samples were acquired by LSR Fortessa (BD Biosciences, Basel, Switzerland), 375 and data analyzed by FlowJo software (Tree Star, Ashland, OR, USA).

### Statistical analysis

Data are presented as mean ± standard error. The significance of differences was assessed with the GraphPad Prism 7.03 software (GraphPad Software). The normal distribution of all data sets was tested and, depending on the results, multiple comparisons were performed with the parametric 1-way analysis of variance (ANOVA) followed by the Sidak test for multiple comparisons, or with the non-parametric Kruskal-Wallis test followed by Dunn's post-test, while single comparisons were analyzed with the non-parametric Mann-Whitney test or the parametric unpaired t-test.

### References as cited/Background art

1. Sellke FW, Laham RJ, Edelman ER, Pearlman JD, Simons M. Therapeutic angiogenesis with basic fibroblast growth factor: Technique and early results. Ann Thorac Surg 1998, 65(6): 1540-1544.
2. Kaushal S, Amiel GE, Guleserian KJ, Shapira OM, Perry 401 T, Sutherland FW, et al. Functional small-diameter neovessels created using endothelial progenitor cells expanded ex vivo. Nat Med 2001, 7(9): 1035-1040.
3. Banfi A, von Degenfeld G, Gianni-Barrera R, Reginato S, Merchant MJ, McDonald DM, et al. Therapeutic angiogenesis due to balanced single-vector delivery of VEGF and PDGF-BB. Faseb J 2012, 26(6): 2486-2497.
4. Isner JM, Walsh K, Symes J, Pieczek A, Takeshita S, Lowry J, et al. Arterial gene therapy for therapeutic angiogenesis in patients with peripheral artery disease. Circulation 1995, 91(11): 2687-2692.
5. Rissanen TT, Vajanto I, Yla-Herttuala S. Gene therapy for therapeutic angiogenesis in critically ischaemic lower limb - on the way to the clinic. Eur J Clin Invest 2001, 31(8): 651-666.
6. Levenberg S, Rouwkema J, Macdonald M, Garfein ES, Kohane DS, Darland DC, et al. Engineering vascularized skeletal muscle tissue. Nat Biotechnol 2005, 23(7): 879-884.
7. Koike N, Fukumura D, Gralla O, Au P, Schechner JS, Jain RK. Tissue engineering: creation of long-lasting blood vessels. Nature 2004, 428(6979): 138-139.
8. Au P, Tam J, Fukumura D, Jain RK. Bone marrow-derived mesenchymal stem cells facilitate engineering of long-lasting functional vasculature. Blood 2008, 111(9): 4551-4558.
9. Avraamides CJ, Garmy-Susini B, Varner JA. Integrins in angiogenesis and lymphangiogenesis. Nat Rev Cancer 2008, 8(8): 604-617.
10. Kim SH, Turnbull J, Guimond S. Extracellular matrix and cell signalling: the dynamic cooperation of integrin, proteoglycan and growth factor receptor. J Endocrinol 2011,209(2): 139-151.
11. Ingber DE. Fibronectin Controls Capillary Endothelial-Cell Growth by Modulating Cell-Shape. P Natl Acad Sci USA 1990, 87(9): 3579-3583.
12. Forget A, Christensen J, Ludeke S, Kohler E, Tobias S, Matloubi M, et al. Polysaccharide hydrogels with tunable stiffness and provasculogenic properties via alpha-helix to beta-sheet switch in secondary structure. P Natl Acad Sci USA 2013, 110(32): 12887-12892.
13. Chen EJ, Novakofski J, Jenkins WK, OBrien WD. Young's modulus measurements of soft tissues with application to elasticity imaging. Ieee T Ultrason Ferr 1996, 43(1): 191-194.
14. Bale MD, Muller MF, Ferry JD. Rheological studies of creep and creep recovery of unligated fibrin clots: comparison of clots prepared with thrombin and ancrod. Biopolymers 1985, 24(3): 461-482.
15. LeBlanc AJ, Krishnan L, Sullivan CJ, Williams SK, 451 Hoying JB. Microvascular repair: post-angiogenesis vascular dynamics. Microcirculation 2012, 19(8): 676-695.
16. Hughes CS, Postovit LM, Lajoie GA. Matrigel: a complex protein mixture required for optimal growth of cell culture. Proteomics 2010, 10(9): 1886-1890.
17. Ponce ML. Tube formation: an in vitro matrigel angiogenesis assay. Methods Mol Biol 2009, 467: 183-188.
18. Malinda KM. In vivo matrigel migration and angiogenesis assay. Methods Mol Biol 2009, 467: 287-294.
19. Ozawa CR, Banfi A, Glazer NL, Thurston G, Springer ML, Kraft PE, et al. Microenvironmental VEGF concentration, not total dose, determines a threshold between normal and aberrant angiogenesis. J Clin Invest 2004, 113(4): 516-527.
20. Potente M, Gerhardt H, Carmeliet P. Basic and therapeutic aspects of angiogenesis. Cell 2011, 146(6): 873-887.
21. Groppa E, Brkic S, Bovo E, Reginato S, Sacchi V, Di Maggio N, et al. VEGF dose regulates vascular stabilization through Semaphorin3A and the Neuropilin-1+ monocyte/TGF-beta1 paracrine axis. EMBO Mol Med 2015, 7(10): 1366-1384.
22. Scholzen T, Gerdes J. The Ki-67 protein: from the known and the unknown. J Cell Physiol 2000, 182(3): 311-322.
23. Gianni-Barrera R, Trani M, Fontanellaz C, Heberer M, Djonov V, Hlushchuk R, et al. VEGF over-expression in skeletal muscle induces angiogenesis by intussusception rather than sprouting. Angiogenesis 2013, 16(1): 123-136.
24. Murdoch C, Muthana M, Coffelt SB, Lewis CE. The role of myeloid cells in the promotion of tumour angiogenesis. Nat Rev Cancer 2008, 8(8): 618-631.
25. Zacchigna S, Pattarini L, Zentilin L, Moimas S, Carrer A, Sinigaglia M, et al. Bone marrow cells recruited through the neuropilin-1 receptor promote arterial formation at the sites of adult neoangiogenesis in mice. J Clin Invest 2008, 118(6): 2062-2075.
26. Blumenthal NR, Hermanson O, Heimrich B, Shastri VP. Stochastic nanoroughness modulates neuron-astrocyte interactions and function via mechanosensing cation channels. Proc Natl Acad Sci U S A 2014, 111(45): 16124-16129.
27. Weis SM, Cheresh DA. alphaV integrins in angiogenesis and cancer. Cold Spring Harb Perspect Med 2011, 1(1): a006478.
28. Nilius B. Pressing and squeezing with Piezos. EMBO Rep 2010, 11(12): 902-903.
29. Li J, Hou B, Tumova S, Muraki K, Bruns A, Ludlow MJ, et al. Piezol integration of vascular architecture with physiological force. Nature 2014, 515(7526): 279-282.
30. Ranade SS, Qiu ZZ, Woo SH, Hur SS, Murthy SE, Cahalan SM, et al. Piezol, a mechanically activated ion channel, is required for vascular development in mice. P Natl Acad Sci USA 2014, 111(28): 10347-10352.

## Claims

1. An isolated mammalian CD11b+/PIEZO-1+ macrophage cell or neutrophil cell.

2. A pharmaceutical composition comprising the CD11b+/PIEZO-1+ macrophage cell or neutrophil cell according to claim 1, together with a pharmaceutically acceptable carrier and/or auxiliary agent.

3. The pharmaceutical composition according to claim 2, further comprising a matrix material selected from the group of alginate, hyaluronic acid, agarose, poloxamers (e.g. Pluronic^{®}), polyethylene (PEG) hydrogels, and a modified primary hydroxyl groups containing polysaccharide comprising repeating disaccharide units, preferably agarose wherein in at least part of the disaccharide units the primary hydroxyl group is replaced by functional groups selected from halide groups or groups comprising sulfur or phosphorus atoms, such as, for example, sulfate groups, sulfonate groups, phosphonate groups and phosphate groups, carboxylated agarose and phosphate agarose.

4. A method for producing a pharmaceutical composition according to claim 2 or 3, comprising the steps of
a) providing a biological sample derived from a mammal comprising CD11b+ macrophages or neutrophil cells, b) purifying and/or isolating CD11b+ PIEZO-1+ macrophages or neutrophil cells from said sample based on the expression of CD11b+ and PIEZO-1+, and
c) formulating said purified and/or isolated CD11b+ PIEZO-1+ macrophages or neutrophil cells into a suitable pharmaceutical composition.

5. The method according to claim 4, wherein said purifying and/or isolating comprises the use of beads, such as Dynal^{®} beads or cell sorting, such as fluorescence activated cell sorting (FACS^{®}).

6. A method for diagnosing angiogenesis in a mammal, comprising identifying CD11b+ PIEZO-1+ macrophages or neutrophil cells in a sample from a mammal to be diagnosed, wherein the presence of CD1 1b+ PIEZO-1+ macrophages or neutrophil cells is indicative for angiogenesis in said mammal, when compared to a control sample.

7. The method according to claim 6, wherein said identifying further comprises detecting the amount of said CD11b+ PIEZO-1+ macrophages and/or neutrophil cells and/or the level of expression and/or the levels of the biological activity of CD1 1b+ and PIEZO-1+ in said macrophages and/or neutrophil cells in said sample, wherein an increase of the amount of said CD11b+ PIEZO-1+ macrophages and/or neutrophil cells and/or the level of expression and/or the levels of the biological activity of CD1 1b+ and PIEZO-1+ in said macrophages and/or neutrophil cells, when compared to a control, is indicative for an increased angiogenesis.

8. A method for identifying a compound that modulates the expression and/or the biological activity of PIEZO-1 in a CD11b+ macrophage and/or neutrophil cell, comprising the steps of a) contacting said CD11b+ macrophage and/or neutrophil cell with at least one compound that potentially modulates the expression and/or the biological activity of PIEZO-1 in said CD11b+ macrophage and/or neutrophil cell, and b) identifying a modulation of the expression and/or the biological activity of PIEZO-1 in the presence of said at least one compound, compared to a control CD11b+ macrophage.

## Patentansprüche

1. Isolierte Säugetier CD1 1b+/PIEZO-1+ Makrophagenzelle oder Neutrophilenzelle.

2. Pharmazeutische Zusammensetzung umfassend die CD11b+/PIEZO-1+ Makrophagenzelle oder Neutrophilenzelle nach Anspruch 1, zusammen mit einem pharmazeutisch akzeptablen Träger und/oder Hilfsstoff.

3. Pharmazeutische Zusammensetzung nach Anspruch 2, weiter umfassend ein Matrixmaterial ausgewählt aus der Gruppe von Alginat, Hyaluronsäure, Agarose, Poloxamere (z.B. Pluronic^{®}), Polyethylen (PEG) Hydrogele und einem modifizierte primäre Hydroxylgruppen enthaltendes Polysaccharid umfassend sich wiederholende Disaccharideinheiten, bevorzugt Agarose, worin zumindest in einem Teil der Disaccharideinheiten die primäre Hydroxylgruppe durch funktionelle Gruppen ausgewählt aus Halogenid-Gruppen oder Gruppen umfassend Schwefel oder Phosphoratome, wie zum Beispiel Sulfatgruppen, Sulfonatgruppen, Phosphonatgruppen und Phosphatgruppen ersetzt sind, carboxylierte Agarose und Phosphatagarose.

4. Verfahren zur Herstellung einer pharmazeutischen Zusammensetzung nach Anspruch 2 oder 3, umfassend die Schritte von
a) zur Verfügung stellen einer biologischen Probe, abgeleitet von einem Säugetier, umfassend CD11b+ Makrophagenzellen oder Neutrophilenzellen, b) Reinigen und/oder Isolieren der CD11b+ PIEZO-1+ Makrophagenzellen oder Neutrophilenzellen aus der Probe basierend auf der Expression von CD1 1b+ und PIEZO-1+, und
c) Formulieren der gereinigten und/oder isolierten CD11b+ PIEZO-1+ Makrophagenzellen oder Neutrophilenzellen in eine geeignete pharmazeutische Zusammensetzung.

5. Verfahren nach Anspruch 4, wobei das Reinigen und/oder Isolieren die Verwendung von Beads, wie zum Beispiel Dynal^{®} Beads oder Cell-sorting, wie zum Beispiel Fluoreszenz aktiviertes Cell-sorting (FACS^{®}) umfasst.

6. Verfahren zur Diagnose von Angiogenese in einem Säugetier, umfassend ein Identifizieren von CD11b+ PIEZO-1+ Makrophagenzellen oder Neutrophilenzellen in einer Probe von einem zu diagnostizierenden Säugetier, wobei die Anwesenheit von CD11b+ PIEZO-1+ Makrophagenzellen oder Neutrophilenzellen wenn verglichen mit einer Kontrollprobe eine Angiogenese in dem Säugetier anzeigt.

7. Verfahren nach Anspruch 6, wobei das Identifizieren weiter ein Nachweisen der Menge an CD11b+ PIEZO-1+ Makrophagenzellen und/oder Neutrophilenzellen und/oder dem Expressionsspiegel und/oder dem Spiegel der biologischen Aktivität von CD11b+ und PIEZO-1+ in den Makrophagenzellen und/oder Neutrophilenzellen in der Probe umfasst, wobei eine Zunahme der Menge an CD11b+ PIEZO-1+ Makrophagenzellen und/oder Neutrophilenzellen und/oder dem Expressionsspiegel und/oder dem Spiegel der biologischen Aktivität von CD11b+ und PIEZO-1+ in den Makrophagenzellen und/oder Neutrophilenzellen, wenn verglichen mit einer Kontrolle, eine erhöhte Angiogenese anzeigt.

8. Verfahren zur Identifizierung einer Verbindung, die die Expression und/oder die biologische Aktivität von PIEZO-1 in einer CD11b+ Makrophagenzelle und/oder Neutrophilenzelle moduliert, umfassend die Schritte von a) in Kontakt bringen der CD11b+ Makrophagenzelle und/oder Neutrophilenzelle mit mindestens einer Verbindung, die potentiell die Expression und/oder die biologische Aktivität von PIEZO-1 in der CD11b+ Makrophagenzelle und/oder Neutrophilenzelle moduliert, und b) Identifizieren einer Modulation der Expression und/oder der biologischen Aktivität von PIEZO-1 in der Anwesenheit der mindestens einen Verbindung, wenn verglichen mit einem Kontroll-CD11b+ Makrophagen.

## Revendications

1. Cellule macrophage CD11b+/PIEZO-1+ ou cellule neutrophile isolée de mammifère

2. Composition pharmaceutique comprenant la cellule macrophage CD11b+/PIEZO-1+ ou la cellule neutrophile selon la revendication 1, avec un support pharmaceutiquement acceptable et/ou un agent auxiliaire.

3. Composition pharmaceutique selon la revendication 2, comprenant en outre un matériau de matrice choisi dans le groupe de l'alginate, de l'acide hyaluronique, de l'agarose, des poloxamères (par ex. Pluronic^{®}), les hydrogels de polyéthylène (PEG), et un polysaccharide contenant des groupes hydroxyle primaires modifiés comprenant des unités disaccharides répétitives, de préférence l'agarose dans lequel dans au moins une partie des unités disaccharides le groupe hydroxyle primaire est remplacé par des groupes fonctionnels choisis parmi les groupes halogénés ou les groupes comprenant des atomes de soufre ou de phosphore, tels que, par exemple, les groupes sulfates, les groupes sulfonates, les groupes phosphonates et les groupes phosphates, l'agarose carboxylé et l'agarose phosphaté.

4. Méthode de production d'une composition pharmaceutique selon la revendication 2 ou 3, comprenant les étapes suivantes
a) fourniture d'un échantillon biologique dérivé d'un mammifère comprenant des cellules macrophages CD11b+ ou neutrophiles,
b) purification et/ou isolation des cellules macrophages CD11b+ PIEZO-1+ ou neutrophiles dudit échantillon sur la base de l'expression de CD11b+ et de PIEZO-1 +, et
c) formulation desdites cellules macrophages CD11b+ PIEZO-1 + ou neutrophiles purifiés et/ou isolés dans une composition pharmaceutique acceptable.

5. Méthode selon la revendication 4, dans laquelle ladite purification et/ou isolation comprend l'utilisation de billes, telles que les billes Dynal^{®} ou le tri cellulaire, tel que le tri cellulaire activé par fluorescence (FACS^{®}).

6. Méthode de diagnostic de l'angiogenèse chez un mammifère, comprenant l'identification des cellules macrophages CD11b+ PIEZO-1+ ou neutrophiles dans un échantillon provenant d'un mammifère à diagnostiquer, dans lequel la présence de cellules macrophages CD11b+ PIEZO-1+ ou neutrophiles est indicative de l'angiogenèse chez ledit mammifère, par rapport à un échantillon témoin.

7. Méthode selon la revendication 6, dans laquelle ladite identification comprend en outre la détection de la quantité desdites cellules macrophages CD11b+ PIEZO-1+ et/ou neutrophiles et/ou du niveau d'expression et/ou des niveaux d'activité biologique de CD11b+ et de PIEZO-1+ dans lesdites cellules macrophages et/ou neutrophiles dans ledit échantillon, dans laquelle une augmentation de la quantité desdites cellules macrophages CD11b+ PIEZO-1+ et/ou neutrophiles et/ou du niveau d'expression et/ou des niveaux d'activité biologique de CD11b+ et de PIEZO-1+ dans lesdits cellules macrophages et/ou neutrophiles, par rapport à un témoin, est indicative d'une angiogenèse accrue.

8. Méthode d'identification d'un composé qui module l'expression et/ou l'activité biologique de PIEZO-1dans une cellule macrophage CD11b+ et/ou neutrophile, comprenant les étapes de a) mise en contact de ladite cellule macrophage CD11b+ et/ou neutrophile avec au moins un composé qui module potentiellement l'expression et/ou l'activité biologique de PIEZO-1dans ladite cellule macrophage CD11b+ et/ou neutrophile, et b) identification d'une modulation de l'expression et/ou de l'activité biologique de PIEZO-1en présence dudit au moins un composé, par rapport à un macrophage CD11b+ témoin.
